# EUROPEAN PATENT SPECIFICATION

(11) **EP 1 929 930 B1**
(45) Date of publication and mention of the grant of the patent: **07.11.2012**
(21) Application number: 06782237.9
(22) Date of filing: 03.08.2006
(51) Int. Cl.: A61B 1/00, A61B 1/04

(54) **ENDOSCOPE APPARATUS**
ENDOSKOPVORRICHTUNG
DISPOSITIF ENDOSCOPE

(30) Priority: 30.09.2005 JP 2005288214
(43) Date of publication of application: 11.06.2008
(73) Proprietor: OLYMPUS MEDICAL SYSTEMS CORP., Tokyo 151-0072 (JP)
(72) Inventor: GONO, Kazuhiro, Tokyo 151-0072 (JP)
(74) Representative: von Hellfeld, Axel
(86) International application number: PCT/JP2006/315377
(87) International publication number: WO 2007/039981

(56) References cited:
- JP-A- 05 084 218
- JP-A- 08 307 890
- JP-A- 2001 299 696
- JP-A- 2003 093 335
- US-A- 5 269 289

## Description

### Technical Field

The present invention relates to an endoscope apparatus suitable for observing a living body to which a photosensitizing substance is sprayed.

### Background Art

In the medical field, a living body is in some cases dyed (colored) to facilitate identifying unevenness, etc. to perform endoscopic observation. As a pigment agent (dyeing agent) for such dyeing, methylene blue is widely used.

For example, in Japanese unexamined patent publication No. 5-84218 as a first exemplary prior art, an endoscope apparatus is disclosed which performs endoscopic observation through dyeing with methylene blue.

Further, in Japanese unexamined patent publication No. 6-339459 as a second exemplary prior art, an endoscope apparatus is disclosed that can perform pigment endoscopy using a pigment. This publication also discloses a function to change light amount of illumination light.

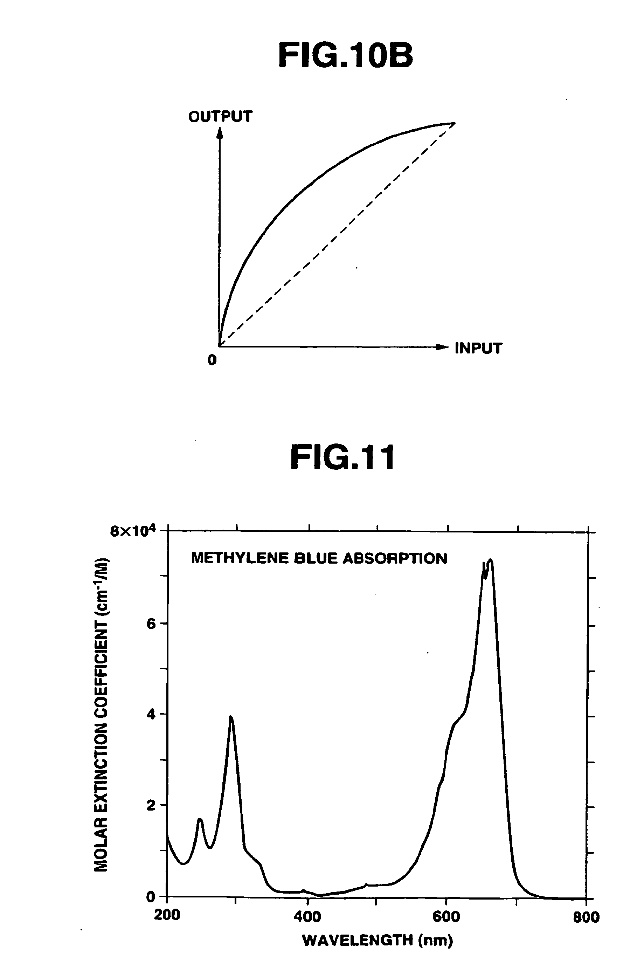

FIG 11 shows exemplary characteristics of methylene blue optical absorption coefficients described in a first non-patent document (Scott Prahl "Optical Absorption of Methylene Blue", "online", "searched on September 12, 2005", Internet <URL:http:/omlc.ogi.edu/spectra/mb/index.html>). As shown in FIG 11, a methylene blue solution has a large absorption peak between near 600 nm and near 700 nm.

The methylene blue mentioned above is known as a photosensitizing substance. As described in the first non-patent document, the methylene blue has sensitiveness to light in a red wavelength band. The methylene blue is excited by irradiation of the light in the red wavelength band to produce reactive oxygen species.

Furthermore, in WO 01/015694 publication as a third exemplary prior art, there is disclosed a method to perform Photo-Dynamic Therapy (PDT) using methylene blue, etc. as a photosensitizing substance.

However, the above-described second exemplary prior art, which discloses an endoscope apparatus that uses methylene blue as a pigment agent, does not disclose changing the amount of the light in the red wavelength range and decreasing or increasing a corresponding color signal on a signal processing apparatus side in response to the change of the light amount.

In the first exemplary prior art, it is disclosed that in the endoscope apparatus which uses methylene blue as a pigment agent, light amount of the red wavelength side is increased and, in response thereto, gain is corrected on a signal processing apparatus side. However, the first exemplary prior art does not at all disclose or suggest light amount control or the like coping with the function of the photosensitizing substance by methylene blue.

In other words, in this first conventional example, though it can be facilitated to identify the unevenness, etc. of an observation target region by the function of the dyeing agent by methylene blue, if the amount of the light in the red wavelength range is increased, reactive oxygen species are also increased because the function of the photosensitizing substance is not taken into consideration.

Moreover, the third exemplary prior art does not disclose performing a signal processing.

US 5,269,289, on which document is based the preamble of claim 1, discloses an endoscope comprising a detection circuit and means for increasing a luminance level of an image that is lowered due to a dye or bodily fluids sticking to the distal end of the endoscope. The detecting means detects which stain is on the endoscope distal end by detecting the hues of the stain.

As such, the exemplary prior arts do not disclose an endoscope apparatus that takes into consideration characteristics of the photosensitizing substance, nor performing a signal processing coping with a light amount control taking into consideration the characteristics of the photosensitizing substance.

The present invention was made in view of the above-mentioned points, and an object of the present invention is to provide an endoscope apparatus having the function of the photosensitizing substance in the red wavelength range such as of methylene blue, and suitable for performing endoscopic observation etc. that takes into consideration the characteristics of the photosensitizing substance.

### Disclosure of Invention

### Means for Solving the Problem

An endoscope apparatus according to independent claim 1 is provided. The endoscope apparatus may include an insertion portion which is insertable into a living body; illumination means which emits illumination light to an observation target region side in the living body; light amount control means which performs light amount control to at least decrease an amount of light in a red wavelength range in the illumination light which excites a photosensitizing substance administered to the observation target region; and signal processing means which, in response to the light amount control to decrease the amount of the light in the red wavelength range, performs signal processing to increase a luminance level of a red color signal that corresponds to a red wavelength range in picking up an image under the illumination light.

With the above-described configuration, when the photosensitizing substance is administered to the observation target region, the amount of the light in the red wavelength region is decreased that serves as an excitation light for the photosensitizing substance and, in response to a control to decrease the light amount, a signal processing is performed to increase a luminance level of the red color signal. Thus, it becomes possible to restrict production of the reactive oxygen species by the photosensitizing substance and to restrict color tone change due to the change of the amount of the light in the red wavelength region, thereby allowing an endoscopic observation to be performed in an appropriate color tone.

### Brief Description of the Drawings

FIG 1 is a block diagram showing an entire configuration of an endoscope apparatus of a first embodiment of the present invention.
FIG 2 is a view showing transmission characteristics of R, G, B filters provided to a rotary filter.
FIG 3A is a timing chart of actions in a first observation mode in the present embodiment.
FIG. 3B is a timing chart of actions in a second observation mode in the present embodiment.
FIG 3C is a timing chart of actions in the second observation mode in the present embodiment.
FIG 4A is a flow chart of workings in the second observation mode in the present embodiment.
FIG 4B is a flow chart of workings in the second observation mode in the present embodiment.
FIG 5 is a block diagram showing an entire configuration of an endoscope apparatus of a second embodiment of the present invention.
FIG 6 is a view showing transmissivity characteristics of a first band-pass filter and a second band-pass filter.
FIG 7 is a block diagram showing a configuration of a light source apparatus in a third embodiment of the present invention.
FIG 8 is a view showing transmissivity characteristics of a third filter.
FIG 9 is a block diagram showing a configuration of a pseudo-R signal generation circuit in a processor.
FIG 10A is a view showing input-output characteristics of a first tone correction circuit.
FIG 10B is a view showing input-output characteristics of a second tone correction circuit.
FIG 11 is a view showing optical absorption characteristics of methylene blue.

### Best Mode for Carrying Out the Invention

Embodiments of the present invention are described below referring to the drawings.

### (First Embodiment)

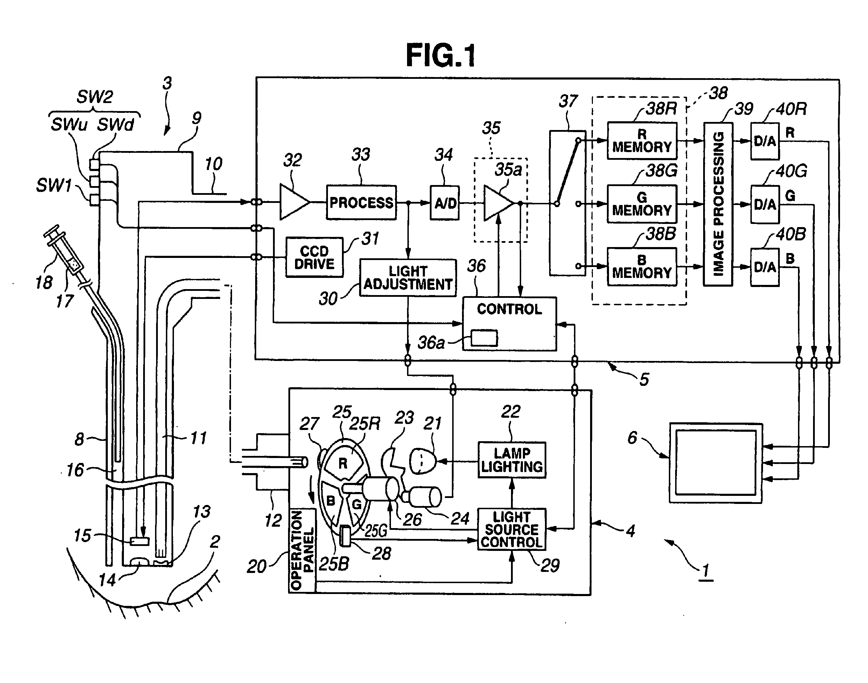

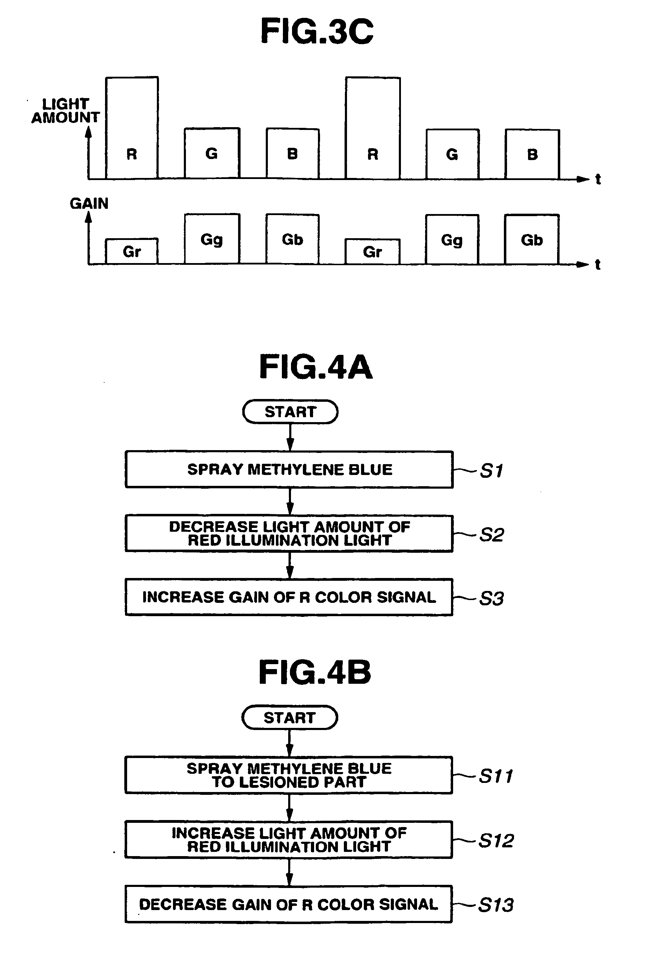

Referring to FIGS. 1 to 4B, a first embodiment of the present invention is described.

As shown in FIG 1, an endoscope apparatus 1 of the first embodiment of the present invention includes: an electronic endoscope (hereinafter abbreviated as scope) 3 which is insertable into a body cavity and picks up an image of an observation target region 2 such as a diseased part in the body cavity to perform an endoscopic observation etc.; a light source apparatus 4 which is detachably connected with the scope 3, for producing illumination light for observation; a processor 5 which is detachably connected with the scope 3, for performing signal processing, etc. on an image picked-up signal; and a monitor 6 which is connected to the processor 5, inputted with a video signal outputted from the processor 5, and displays an image corresponding to this video signal.

The scope 3 includes an elongate insertion portion 8 which is inserted into the body cavity, an operation portion 9 provided at a rear end of the insertion portion 8, and a universal cord 10 extended from the operation portion 9.

In the insertion portion 8 of the scope 3 is inserted a light guide fiber 11 for transmitting illumination light and a rear end side of the light guide fiber 11 is inserted in through the universal cord 10. The light guide fiber 11 has at a rear end thereof a light guide connector 12 which is detachably connected to the light source apparatus 4, thus allowing illumination light to be supplied from the light source apparatus 4.

The illumination light transmitted by the light guide fiber 11 is emitted from a distal end surface mounted to an illumination window of a distal end portion of the insertion portion 8, further through an illumination lens 13, to the observation target region 2 side in the body cavity.

Provided adjacent to the illumination window is an observation window (image pickup window) to which an object lens 14 is mounted. At an image forming position of the object lens 14, a solid-state image pickup device, namely, a CCD15 is arranged to photoelectrically convert an optical image formed on the image pickup surface. The CCD15 has a connector at an end portion thereof which is detachably connected to the processor 5 through a signal line.

The insertion portion 8 of the scope 3 is provided with a channel 16 through which a treatment instrument or the like is insertable. In the present embodiment, through the channel 16 is inserted a syringe 18 containing a solution of methylene blue 17 that has a dyeing function of a pigment agent and a photosensitizing function, so as to administer (more specifically spray) the solution of the methylene blue 17 to the observation target region 2 to allow an observation thereof to be performed.

As shown in FIG 11, the methylene blue 17 has optical absorption peaks in a red wavelength range between near 600 nm and near 700 nm (more specifically, a maximum peak at 668 nm and a second peak at 609 nm). This optical absorption provides a photosensitive function to produce reactive oxygen species, more specifically a photosensitizing function (as a substance).

That is, it can be said that the methylene blue 17 is a photosensitizing substance to produce reactive oxygen species, excited by the light in the wavelength range of the optical absorption peak serving as an excitation light.

In the present embodiment, observation can be performed in, other than a normal observation mode (hereinafter referred to as first observation mode) by normal frame sequential illumination light, a dyeing or photosensitization observation mode (hereinafter referred to as second observation mode) which is suitable for when the methylene blue 17 is sprayed (administered). To the operation portion 9, for example, of the scope 3, there are provided with a mode change-over switch SW1 for changing over between first and second observation modes, and a light amount change switch SW2 which, when the mode change-over switch SW1 is changed over to the second observation mode, causes a state suitable for the dyeing or photosensitizing function and changes an amount of light in the red wavelength band so as to allow a user to easily make a change setting.

Note that the light amount change switch SW2 includes a light amount up switch SWu for increasing the amount of the light in the red wavelength band and a light amount down switch SWd for decreasing (turning down) the light amount.

The light source apparatus 4 incorporates a lamp 21 such as a xenon lamp that emits a light that covers a visible range. The lamp 21 can change light emission amount thereof through lamp lighting electric power from a lamp lighting circuit 22.

On an illumination light path of the lamp 21 is provided a diaphragm 23 for increasing and decreasing the amount of light passing the same. The diaphragm 23 changes an opening amount thereof by a diaphragm motor 24, thereby increasing and decreasing the amount of light passing the amount of opening.

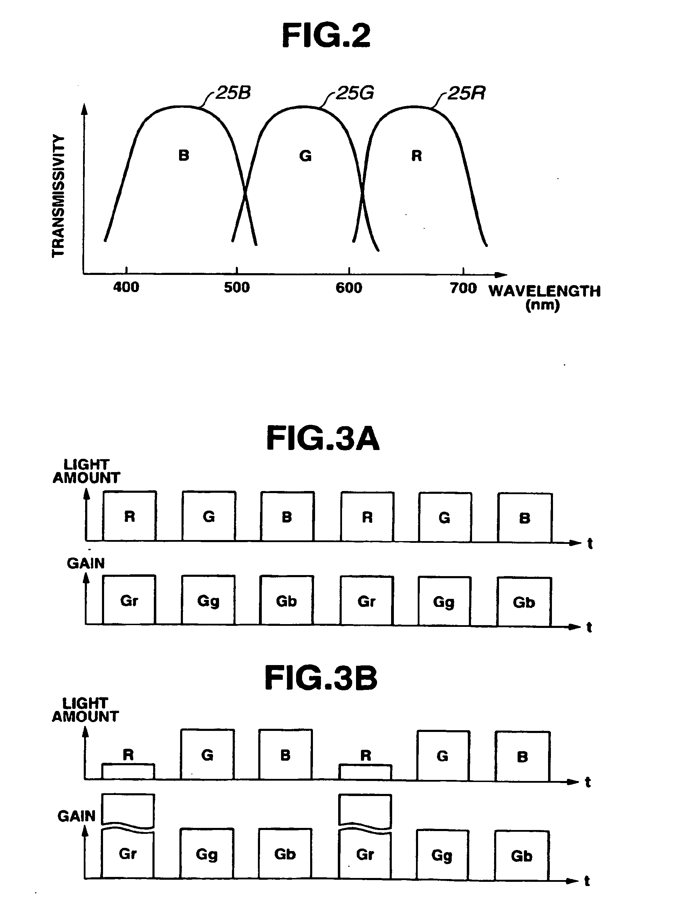

The light that passed the diaphragm 23 is incident into a rotary filter 25. In a circumferential direction of the rotary filter 25, there are provided R, G, B filters 25R, 25G 25B to transmit lights in red (R), green (G), and blue (B) wavelength ranges, respectively, covering a visible range. FIG 2 shows transmission characteristics of the R, G, B filters 25R, 25G, 25B. As shown in FIG 2, the R, G, B filters 25R, 25G, 25B pass the R, G, B wavelength ranges, respectively, in a wide band.

The rotary filter 25 is rotationally driven at a constant speed by a motor 26, to sequentially arrange the R, G, B filters 25R, 25G, 25B in the light path. R, G, B illumination lights that transmitted the filters arranged in the light path in the rotary filter 25 are condensed by a condensing lens 27 to be sequentially incident on a rear end surface of the light guide fiber 11 in a time divisional manner. In other words, the light source apparatus 4 in the present embodiment produces frame sequential illumination lights.

Close to the rotary filter 25 is provided a sensor 28 for detecting a rotational position of the rotary filter 25. The sensor 28 detects which filter is arranged in the light path and outputs a detection result to the light source control circuit 29.

The light source control circuit 29 controls rotational speed of the motor 26 by an output signal of the sensor 28, and controls to change the lamp lighting electric power of the lamp lighting circuit 22 in response to an instruction operation of the light amount change switch SW2. That is, the light source control circuit 29 increases or decreases the light emission amount of the lamp 21 at a timing when the R filter 25R is arranged in the light path. Note that the diaphragm motor 24 adjusts the opening amount of the diaphragm 23 by a light adjusting signal from a light adjusting circuit 30 of the processor 5.

Note that a user can perform an instruction operation similar to that with the light amount change switch SW2, etc. also by operating an operation panel 20 provided to the light source apparatus 4.

On the other hand, the processor 5 incorporates a CCD driving circuit 31. A CCD drive signal produced by the CCD driving circuit 31 is applied to the CCD15. When applied with the CCD drive signal, the CCD 15 picks up an image under the R, Q B frame sequential illumination lights and sequentially outputs photoelectrically converted CCD output signals, that is, R, G, B signals.

The R, G, B signals are inputted to an amplifier 32 in the processor 5 to be amplified and then inputted to a processing circuit 33 to be subjected to CDS processing, etc. Then, the R, Q B signals are inputted to an AD conversion circuit 34 to be converted from analog to digital signals, and thereafter to a gain variable amplifier 35a configuring a white balance circuit 35.

Output signal of the processing circuit 33 is also inputted to the light adjusting circuit 30 for generating the light adjusting signal, so as to generate the light adjusting signal. The light adjusting signal then adjusts the opening amount of the diaphragm 23 through the diaphragm motor 24.

Output signal of the gain variable amplifier 35a is inputted to the control circuit 36 configuring light amount control means and at the same time sequentially stored in R, Q B memories 38R, 38G, 38B configuring a coincidence circuit 38, through a selector 37.

In white balance adjustment, the control circuit 36 takes in the output signal of the gain variable amplifier 35a and adjusts a gain value of the gain variable amplifier 35a by a gain control voltage so as to perform white balance. In other words, the control circuit 36 adjusts the gain value of the gain variable amplifier 35a by the gain control voltage applied to a gain control end of the gain variable amplifier 35a.

After the white balance adjustment, the gain control voltage is applied to the gain variable amplifier 35a at a timing when R, G, B color signals are inputted thereto, so as to maintain the white balance state.

In the second observation mode, the control circuit 36 sends to the light source control circuit 29 a signal corresponding to the instruction operation of the light amount change switch SW2. The light source control circuit 29 causes the lamp lighting circuit 22 to change the lamp lighting electric power at a timing when the R filter 25R is arranged in the light path in response to the instruction operation of the light amount change switch SW2, thereby controlling to change the light amount of the red illumination light.

In the second observation mode, along with the change of the light amount of the red illumination light by sending the signal corresponding to the instruction operation of the light amount change switch SW2 to the light source control circuit 29 as mentioned above, the control circuit 36 also changes, in synchronization with the change of the light amount of the red illumination light, the gain of the R color signal of an image picked up in an illumination state under the changed amount of the red illumination light.

In this case, the control circuit 36 includes inside thereof an EEPROM 36a, for example, as a nonvolatile memory storing information of a look-up table for setting the gain of the gain variable amplifier 35a to an arbitrary value. The look-up table stores, for example, information to associate a gain control voltage and a gain to be set by this gain control voltage.

When the light amount of the red illumination light is set to be changed, the control circuit 36 refers to a gain value under the light amount state of the red illumination light before the change, depending on the indicated value of the change setting. Thus, a gain change setting is made such that the white balance state is maintained even if the light amount of the red illumination light is changed.

Thus, in the present embodiment, the signal processing in the processor 5 is controlled so as to, even if the light amount of the red illumination light is changed, restrict color tone change of an observation image caused by the light amount change of the red illumination light. In other words, the present embodiment is configured such that the signal processing in the processor 5 is controlled such that, even if the light amount of the red illumination light is changed, the white balance state before the change of the light amount is maintained.

As discussed above, the output signal of the gain variable amplifier 35a is sequentially stored in the R, G, B memories 38R, 38G, 38B configuring the coincidence circuit 38, through the selector 37.

In other words, the R, G, B color signals of an image picked up under the R, G, B illumination lights are sequentially stored in the R, G, B memories 38R, 38G, 38B, respectively.

The R, G, B color signals stored in the R, G, B memories 38R, 38G, 38B are simultaneously read, inputted to an image processing circuit 39 to be subjected to image processings such as gamma correction and outline emphasis, and then converted into analog color signals by D/A conversion circuits 40R, 40G, 40B. The converted color signals are outputted to the monitor 6, so that an image picked up by the CCD15 is color-displayed on a display surface of the monitor 6.

The following describes workings by the present embodiment having such configuration.

Before the scope 3 is inserted into the body cavity to perform endoscopy, white balance adjustment is performed using a white subject not shown. Setting is made for picking up an image of the white subject and a white balance adjustment switch not shown is operated.

Then, R, G, B color signals picked up of the image of the white subject illuminated with the R, G, B frame sequential illumination lights are inputted to the gain variable amplifier 35a. Output signal of the gain variable amplifier 35a is taken into the control circuit 36. In this initial state, the control circuit 36 stores a mean value of luminance levels of the R, G, B color signals in the EEPROM 36a in the control circuit 36, in a state where, for example, the gain of the gain variable amplifier 35a is set to 1.

Next, from the mean value of the luminance levels of the R, G, B color signals stored in the EEPROM 36a, the control circuit 36 controls gains Gr, Gg, Gb of the gain variable amplifier 35a by the gain control voltage such that R, G, B color signals outputted from the gain variable amplifier 35a have a uniform illuminance level.

In other words, the control circuit 36 applies the gain control voltage to the gain variable amplifier 35a at the timing when the R, G, B color signals are inputted to the gain variable amplifier 35a, such that the gains Gr, Gg, Gb are set to a white balance state where the R, G, B color signals outputted from the gain variable amplifier 35a have a uniform luminance level. Note that the control circuit 36 stores and maintain in the EEPROM 36a the values of the gains Gr, Gg, Gb (or gain control voltage) obtained after the adjustment to the white balance state.

Also note that, when adjusting the white balance, one color signal may be referenced to adjust the other two color signals. In other words, one of the three gains may be fixed to a reference value and the remaining two values be variably controlled.

After the white balance setting is thus completed, endoscopy is performed. The normal observation is performed in the first observation mode. In the first observation mode, the R, G, B filters 25R, 25G, 25B of the rotary filter 25 are sequentially arranged in the illumination light path in a manner shown in the upper side of FIG 3A, so that R, G, B illuminations are sequentially performed.

The gains Gr, Gg, Gb in this state are shown in the lower side of FIG 3A.

FIG 3A shows a case where the gains Gr, Gg, Gb in the first observation mode are the same for the sake of simplification (for easy understanding of the actions in the second observation mode corresponding to those in the first observation mode).

In the first observation mode, even when a light adjusting function is brought into action, the light amounts of the R, G, B illumination lights are simultaneously changed. Therefore, relative relationship between the upper and lower sides of FIG 3A becomes the same. Note that horizontal axes t in FIGS. 3A to 3C show time.

Meanwhile, the operator may in some cases desire to observe the observation target region 2 by facilitating it to better identify the condition of unevenness of the region by using the function of the dyeing agent of the methylene blue 17 sprayed to the region.

In this case, the operator inserts a tube of the syringe 18 into the channel 16 as shown in FIG 1 and further protrudes a distal end side of the tube from a distal end aperture of the channel 16 to spray (administer) the methylene blue 17 to the observation target region 2. The methylene blue 17 sprayed accumulates according to the condition to the unevenness of the surface of the observation target region 2, the dyeing density and optical absorption intensity of the accumulation facilitating it to better identify the unevenness condition.

In this case, the operator decreases the light amount of the red illumination light by operating the light amount down switch SWd of the light amount change switch SW2.

By operating the light amount down switch SWd, the light amount of the red illumination light becomes smaller as shown in the upper side of FIG 3B. In synchronization therewith, the control circuit 36 increases the gain Gr for the R color signal as shown in the lower side of FIG 3B, thereby maintaining the white balance state. FIG 3B shows a case where the light amount of, for example, the red illumination light is one third of that of FIG 3A, so that the gain Gr for the R color signal shown in FIG 3B becomes three times the gain Gr of FIG 3A.

More generally, if the light amount value of the red illumination light in the state of FIG 3A is assumed as 1 and this value is changed to be set to, for example, a light amount value Qr, then the gain value for the R color signal in this case is set to a gain value in inverse proportion to the light amount value Qr.

Thus, by decreasing the light amount of the red illumination light that serves as an excitation light to cause the methylene blue 17 to produce the reactive oxygen species, it becomes possible to restrict the production amount of the reactive oxygen species, facilitate identifying the condition of the unevenness of the observation target region 2, and further perform an observation while maintaining the white balance state.

In other words, because the production amount of the reactive oxygen species by the methylene blue 17 is restricted compared to those in the exemplary prior arts, it is enabled to reduce the influence on the observation target region 2 by the reactive oxygen species produced by the methylene blue 17, which permits conducting an observation (diagnosis) in a more desirable state.

Meanwhile, if the observation target region 2 is a lesioned part, for example, and the lesioned part is sufficiently identifiable, then it is also possible to perform a treatment to conduct Photo-Dynamic Therapy (PDT) on the lesioned part by the reactive oxygen species, using the photosensitizing function of the methylene blue 17 that is sprayed (administered) to the lesioned part. In other words, by using the photosensitizing function of the methylene blue 17, the methylene blue 17 can be used as a medicine.

In this case, the operator increases the light amount of the red illumination light as shown in the upper side of FIG 3C by operating the light amount up switch SWu. This operation also renders the gain Gr for the R color signal smaller as shown in the lower side of FIG 3C, thereby maintaining the white balance state.

The increase of the light amount of the red illumination light allows the methylene blue 17 sprayed to the lesioned part to absorb the light to increase the production amount of the reactive oxygen species, causing the produced reactive oxygen species to work as a medicine that efficiently works on the lesioned part for therapy thereof.

Also in this case, the white balance state is maintained, which allows a display maintaining a normal color tone. Thus, corruption of the color tone as an important factor for endoscopy can be prevented, allowing the operator to smoothly perform endoscopy.

Flow chart representations of the activities in FIGS. 3B and 3C are as shown in FIGS. 4A and 4B, respectively. When observing the observation target region 2 using the dyeing agent function by the methylene blue 17 as to facilitate better identifying the condition of the unevenness of the observation target region 2, the operator proceeds as shown in FIG 4A.

That is, as shown in step S1, the operator sprays the methylene blue 17 to the surface of the observation target region 2.

In the next step S2, the operator operates the light amount down switch SWd. This causes the control circuit 36 to decrease the light emission amount of the lamp 21 in emitting the red illumination light, through the light source control circuit 29 of the light source apparatus 4. Thus, even if the red illumination light is irradiated to the methylene blue 17, the production rate of the reactive oxygen species by the photosensitizing function of the methylene blue 17 can be restricted.

Interlockingly with the operation of step S2, the control circuit 36 increases the gain for the R color signal as shown in step S3 to maintain the white balance state. Thus, the natural color tone can be ensured.

On the other hand, when performing on the observation target region 2 a medicinal therapeutic treatment using the photosensitizing function by the methylene blue 17, the operator proceeds as shown in FIG 4B.

As shown in step S 11, the operator sprays the methylene blue 17 to the lesioned part of the observation target region 2.

In the next step S12, the operator operates the light amount up switch SWu. This causes the control circuit 36 to increase the light emission amount of the lamp 21 in emitting the red illumination light, through the light source control circuit 29 of the light source apparatus 4. Thus, by the photosensitizing function when the red illumination light is irradiated to the methylene blue 17, the production of the reactive oxygen species can be increased. The lesioned part sprayed with the methylene blue 17 can then be subjected to therapy by the reactive oxygen species.

Interlockingly with the operation of step S12, the control circuit 36 decreases the gain for the R color signal as shown in step S13 to maintain the white balance state. Thus, the natural color tone can be ensured.

Thus, according to the present embodiment, when conducting an observation or treatment using the methylene blue 17, illumination and signal processing are performed in consideration of the characteristics and function of the photosensitization by the methylene blue 17. This allows effectively using the characteristics and function of photosensitization by the methylene blue 17 for more appropriate observation, treatment, etc. than in the exemplary prior arts.

### (Second Embodiment)

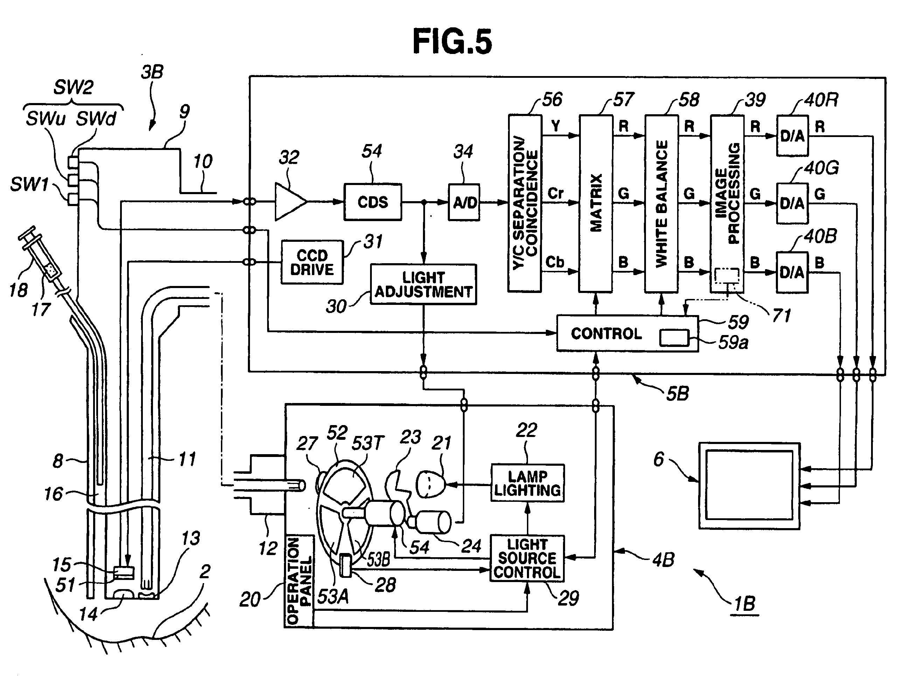

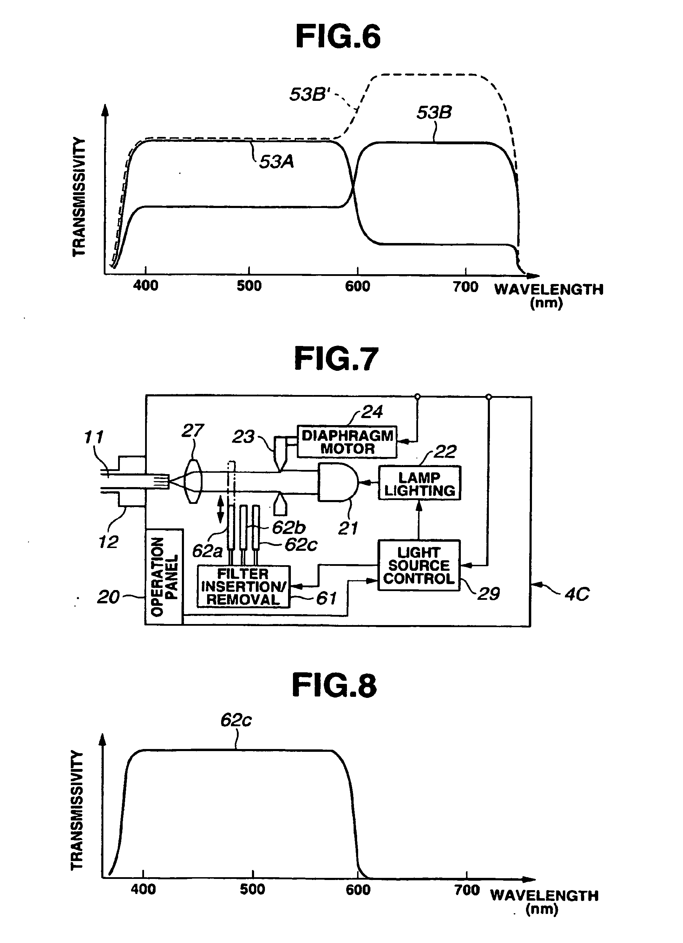

Next, a second embodiment of the present invention is described referring to FIGS. 5 and 6. FIG 5 shows a configuration of a coincidence-type endoscope apparatus 1B of the second embodiment.

The endoscope apparatus 1B includes a scope 3B, a light source apparatus 4B, a processor 5B, and the monitor 6.

The scope 3B includes a CCD for picking up a color image wherein the image pickup surface of the CCD15 in the scope 3 of FIG 1 is provided with a color separation filter 51. In other words, the scope 3B is a coincidence-type scope. As in the first embodiment, the operation portion 9 of the scope 3B is provided with the mode change-over switch SW1 for changing over to the first mode which corresponds to the normal observation, the light amount up switch SWu for performing an instruction operation to increase the light amount of the red illumination light in the second observation mode, and the light amount down switch SWd for reducing the light amount. Here, as in the first embodiment, the light amount up switch SWu and the light amount down switch SWd are generally denominated as the light amount change switch SW2.

The light source apparatus 4B is configured to exclude the rotary filter 25 from the light source apparatus 4 of FIG 1, such that a transparent portion 53T provided to a filter plate 52 is inserted (arranged) in the illumination light path when the mode change-over switch SW1 is operated.

The filter plate 52 is provided in the circumferential direction of a rotation plate thereof with, in addition to the transparent portion 53T, a first band-pass filter 53A and a second band-pass filter 53B. By rotating the filter plate 52 by a motor 54 by a predetermined angle, the first band-pass filter 53A or the second band-pass filter 53B can be arranged in the light path from the state where the transparent portion 53T is arranged in the light path.

FIG 6 shows transmissivity characteristics of the first band-pass filter 53A and the second band-pass filter 53B.

As shown in FIG 6, the first band-pass filter 53A has characteristics to pass lights in blue and green wavelength ranges and restrict transmission of the light in the red wavelength range that serves as a characteristic optical absorption range of the methylene blue. The second band-pass filter 53B has a characteristic to transmit the light in the red wavelength range more than the lights in the blue and green wavelength ranges.

When the first observation mode is set through the mode change-over switch SW1, the transparent portion 53T is arranged in the light path as shown in FIG 5. Note that the transparent portion 53T has a characteristic of being transparent (uniform characteristic) to all wavelength ranges like that of an aperture.

When the light amount down switch SWd is operated, the first band-pass filter 53A is arranged in the light path.

When the light amount up switch SWu is operated, the second band-pass filter 53B is arranged in the light path. In this case, the light source control circuit 29 further causes the lamp lighting circuit 22 to increase the lamp lighting electric power to make a setting as shown in a transmission characteristic 53B' in dotted line of FIG 6.

The transmission characteristic 53B' in dotted line is a characteristic to keep light amounts of the illumination lights in blue and green wavelength ranges same as the amounts of the lights passing through the transparent portion 53T, while significantly increasing the light amount of the red illumination light.

The processor 5B in the present embodiment includes the amplifier 32 for amplifying the output signal of the CCD15. An output signal of the amplifier 32 is inputted to the AD conversion circuit 34 and the light adjusting circuit 30 via a CDS circuit 54.

An output signal of the AD conversion circuit 34 is inputted to a Y/C separating/coincidence circuit 56, and the Y/C separating/coincidence circuit 56 generates a luminance signal Y and coincided color difference signals Cr/Cb.

The output signals of the Y/C separating/coincidence circuit 56 are inputted to a matrix circuit 57, in which the luminance signal Y and the color difference signals Cr/Cb are converted to RGB signals.

The output signals of the matrix circuit 57 are inputted to a white balance circuit 58 to be subjected to white balance processing, and then outputted to the monitor 6 via the image processing circuit 39 and the D/A conversion circuits 40B to 40R as in the first embodiment.

A signal caused by an instruction operation through the light amount change switch SW2, etc. of the scope 3B is inputted to the control circuit 59. The control circuit 59 controls the matrix circuit 57 and the white balance circuit 58.

That is, the control circuit 59 communicates with the light source control circuit 29 of the light source apparatus 4B. In the first observation mode as the normal observation, the control circuit 59 causes the white balance circuit 58 to perform white balance adjustment that corresponds to the normal illumination state where the transparent portion 53T is arranged in the illumination light path, and causes the matrix circuit 57 to perform matrix conversion.

In response to the instruction operation through the light amount change switch SW2 of the scope 3B, the control circuit 59 changes a matrix coefficient for conversion from the signals Y, Cr, Cb to R, G, B in the matrix circuit 57, so as to maintain the white balance state even if the light amount of the red illumination light is increased or decreased.

To this end, the control circuit 59 includes, for example, an EEPROM 59a storing, in addition to information on conversion matrix coefficient for when the transparent portion 53T is set in the light path, information on matrix coefficients for when the first band-pass filter 53A and the second band-pass filter 53B are set in the light path.

A working of the present embodiment thus configured is described. The working by the present embodiment is almost the same as that of when the R, G, B illumination which is frame sequentially performed in the first embodiment is simultaneously performed.

In other words, in the first observation mode, the light source apparatus 4B emits white illumination light by the lamp 21, so that the observation target region 2 is illuminated with the white light.

The observation target region 2 thus illuminated is picked up in a color image by the CCD15 provided with the color separation filter 51. An output signal of the CCD15 is subjected to signal processing by the processor 5B and color-displayed by the monitor 6. In this case, if white balance adjustment is conducted in advance, a white subject is displayed in white.

When observing the observation target region 2 side that is sprayed with the methylene blue 17 to be dyed to facilitate identifying the unevenness condition of the region, it is only necessary to operate the light amount down switch SWd as described in the first embodiment. By performing this operation, the first band-pass filter 53A is arranged in the light path and the light amount of the red illumination light is decreased. This brings the photosensitizing function of the methylene blue 17 to a restricted state. In other words, the production of the reactive oxygen species is restricted.

Furthermore in this case, gain for the R color signal is increased to maintain the white balance state, thus allowing an observation in an appropriate color tone.

Meanwhile, when performing therapy on the lesioned part in the observation target region 2 by the photosensitizing function of the methylene blue 17 administered to the lesioned part, it is only necessary to operate the light amount up switch SWu as described in the first embodiment. By performing this operation, the second band-pass filter 53B is arranged in the light path and the light amount of the red illumination light is increased. Thus, it is made possible to perform therapy on the lesioned part by the reactive oxygen species caused by the photosensitizing function of the methylene blue 17 administered to the lesioned part.

As such, the present embodiment has an effect similar to that in the first embodiment.

### (Third Embodiment)

Next, a third embodiment of the present invention is described referring to FIG 7. The third embodiment has a configuration where, for example, a part of the second embodiment is modified. FIG 7 shows a configuration of a light source apparatus 4C in the third embodiment. The light source apparatus 4C is configured such that, in place of the filter plate 52 provided in, e.g., the light source apparatus 4B in FIG 5, a filter inserting/removing apparatus 61 selectively arranges one of a plurality of filters 62a to 62c in the illumination light path.

Action of the filter inserting/removing apparatus 61 is controlled by the light source control circuit 29. In the present embodiment, a state where none of the filters are arranged in the illumination light path corresponds to the state in the second embodiment where the transparent portion 53T is arranged in the illumination light path, that is, the normal observation.

A state where the first filter 62a or the second filter 62b is arranged in the illumination light path by the filter inserting/removing apparatus 61 corresponds to the state in the second embodiment where the first band-pass filter 53A or the second band-pass filter 53B is arranged in the illumination light path.

Therefore, the present embodiment realizes the same functions as in the second embodiment except for the modified configuration such that the filter is insertably and removably arranged in the illumination light path, a processor 5C of the present embodiment having the functions of the processor 5B of the second embodiment.

In the present embodiment, a third filter 62c can be further arranged in the illumination light path. The third filter 62c has transmission characteristics shown in FIG 8, for example. The third filter 62c has a characteristic to allow substantially no transmission of light in the red wavelength range in the transmission characteristics of the first band-pass filter 53A.

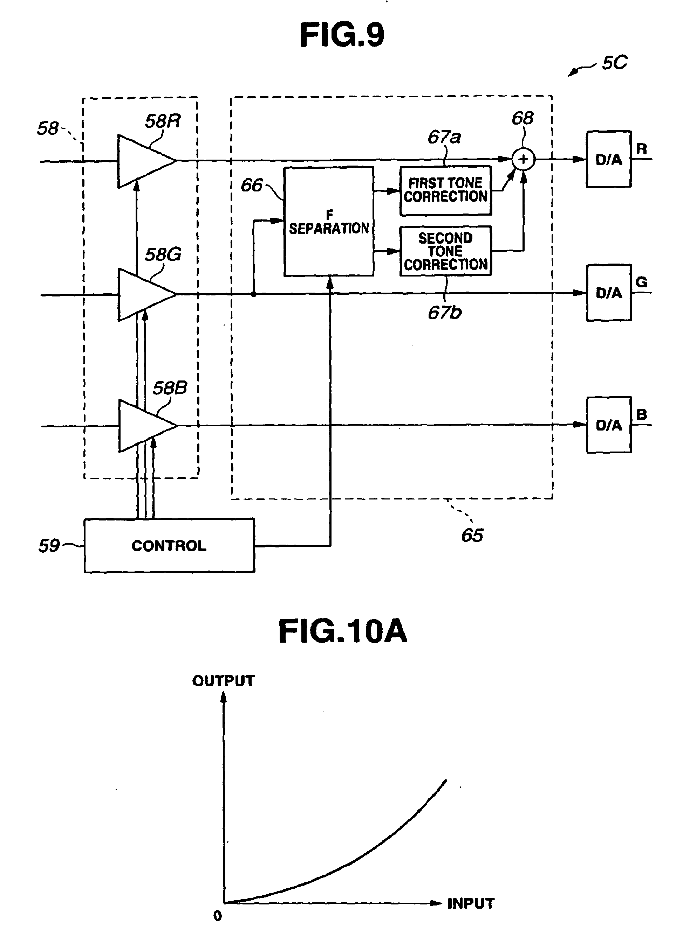

Accordingly, the processor 5C of the present embodiment is designed such that the image processing circuit 39 for performing image processings such as outline emphasis in the processor 5B of the second embodiment further includes a pseudo-R signal generation circuit 65 shown in FIG 9. The processor 5C is configured to generate in a pseudo-manner an R signal from a G signal, for example. Note that FIG 9 only shows a main part in the processor 5C.

Note that the scope in the present embodiment is designed such that the scope 3B of the second embodiment further includes a switch SWc (here referred to as a red cut switch) to be operated to arrange the third filter 62c in the illumination light path. In addition to this example, the light amount down switch SWd may be configured, for example, to function as a red cut switch when operated several times.

As shown in FIG 9, the white balance circuit 58 includes amplifiers 58R, 58G, 58B. When the red cut switch SWc is operated, the control circuit 59 sets the pseudo-R signal generation circuit 65 to an action state, causing a G signal outputted from the amplifier 58G to be inputted to the pseudo-R signal generation circuit 65 so as to generate an R signal in a pseudo-manner.

The G signal inputted to the pseudo-R signal generation circuit 65 passes through the same as-is as the G signal to be inputted to the D/A conversion circuit 40G, while at the same time inputted to a spatial frequency separation circuit 66 (abbreviated simply as F separation in FIG 9). The spatial frequency separation circuit 66 separates an input signal to frequency components higher and those lower than a predetermined spatial frequency as a border.

This predetermined spatial frequency is set to a value between, for example, a spatial frequency that characteristically represents an outline of thin blood vessels running near a mucous membrane surface and a spatial frequency that characteristically presents an outline of thicker blood vessels running on a deeper side than the thin blood vessels. Note that the spatial frequency separation circuit 66 can be configured by a high pass filter and a low pass filter.

A signal on a high frequency side that is outputted from the spatial frequency separation circuit 66 is subject to a first tone correction by a first tone correction circuit 67a, and then inputted to an adder 68 to which an output signal of the amplifier 58R is inputted, to be added therewith. The resulting signal is inputted as an R signal to the D/A conversion circuit 40R.

A signal on a low frequency side that is outputted from the spatial frequency separation circuit 66 is subject to a second tone correction by a second tone correction circuit 67b, to be inputted to the adder 68 to which an output signal of the amplifier 58R is inputted, to be added therewith. The resulting signal is inputted as an R signal to the D/A conversion circuit 40R.

FIGS. 10A and 10B show gradation characteristics of the first and second tone correction circuit 67a, 67b, respectively. The first tone correction circuit 67a is set to a characteristic that restricts the gradation of the output value with respect to an input value, thus restricting the illuminance value of the signal on the high frequency side. In contrast, the second tone correction circuit 67b is set to a characteristic that increases the gradation of the output value with respect to an input value, thus increasing the illuminance value of the signal on the low frequency side.

Therefore, it is made possible to display in red in a pseudo manner an image picked up of the outline of thicker blood vessels running on the deeper side than the living body surface layer as if the blood vessels were observed with an actual light in the red wavelength range when observing a living body mucous membrane. For this reason, even if the red wavelength range is not used in the illumination light, an image can be displayed in a natural color tone as if the red wavelength range were used in the illumination light.

Accordingly, according to the present embodiment, even if the light amount of the red illumination light is further reduced and cut in the second embodiment, an image can be functionally displayed in a natural color tone. Besides the above, workings and effects of the present embodiment are the same as in the second embodiment. Note that, although an example has been described of generating the R color signal from the G color signal, the R color signal may be generated from both the G and B color signals or from the B color signal.

Also note that although the present embodiment has described the configuration and working of cutting the light amount of the red illumination light in the coincidence-type, the configuration and working may also be applied to the frame sequential type. In this case, it is only necessary to provide the pseudo-R signal generation circuit 65 shown in FIG 9 to the image processing circuit 39 in FIG 1, for example.

In addition, although the present embodiment is configured such that the three filters 62a to 62c are freely insertable in and removable from the illumination light path, the number of the insertable and removable filters may be further increased, etc., to render selectable and settable a filter having a more appropriate characteristic.

Note that in each of the above-described embodiments, there may be provided detecting means for detecting whether or not a photosensitizing substance such as the methylene blue 17 or the like is administered on the observation target region 2 side, for example.

Through signal processing by a detecting portion (symbol 71 in double dotted line) in, e.g., the image processing circuit 39 in, e.g., the processor 5B in FIG 5, it is detected whether or not a predetermined value or more of pixels are detected in the blue color signal since the methylene blue 17 has the function of the blue pigment agent. If such pixels are detected, the detecting portion 71 sends a detection signal thereof to the control circuit 59.

This detection signal may cause the control circuit 59 to automatically perform an automatic (red) light amount control for decreasing the red light amount (as if the light amount down switch SWd were manually operated). Along with the decrease, the gain of the red color signal is increased.

Such a configuration allows that, when the methylene blue 17 is sprayed on the observation target region 2 side, the red illumination light is automatically decreased, normally, so as to automatically restrict the production amount of the reactive oxygen species by the photosensitizing substance by the methylene blue 17, to observe the observation target region 2. This eliminates the need for the operator to manually operate to decrease the red light amount, which can improve operability.

When performing the photo-dynamic therapy administering the methylene blue 17 intensively to the lesioned part, by canceling the setting of the automatic control of the (red) light amount to allow manually increasing the red light amount to conduct a treatment, the photo-dynamic therapy can be adequately addressed. In other words, it may be enabled to change over from the mode of performing the light amount control which automatically decreases the red light amount to the manually operated light amount control.

Note that, the specific example, etc. described in the case of FIG 5, of providing the detecting means for detecting whether or not a photosensitizing substance such as the methylene blue 17 is administered on the observation target region 2 side, may also be applied to other embodiments. Note that, although the lamp 21 such as the xenon lamp is used as a light source for illumination in each of the above-described embodiments, no limitation is placed thereon. A laser diode and a light emitting diode (LED) may also be used as a light source.

For example, when using the LED, the LED may be provided to the distal end portion of the insertion portion 8 to emit illumination light to the observation target region 2 side without using the light guide fiber 11. Embodiments, etc. configured by partial combination or the like of the above-described embodiments also belong to the present invention.

### Industrial Applicability

If a region in the body cavity to be observed by an endoscope has both functions of dyeing and photosensitization of the methylene blue or the like, the amount of the light in the red wavelength range as the excitation light of photosensitization is restricted in consideration of the photosensitization function to facilitate identifying unevenness by the dyeing. The case of performing photo-dynamic therapy can also be adequately addressed.

## Claims

1. An endoscope apparatus (1B) comprising:
an endoscope (3B) including an insertion portion (8) which is insertable into a living body; and
illumination means (4) which is configured to emit illumination light to an observation target region side in the living body;
**characterized by** further comprising:
light amount control means (29) configured to control an amount of light in a red wavelength range in the illumination light which causes production of reactive oxygen species by a photosensitizing substance (17); and
signal processing means (5, 5B, 5C) which, in response to the light amount control of the red wavelength range, is configured to perform signal processing to change a luminance level of a red color signal that corresponds to a red wavelength range in picking up an image under the illumination light.

2. The endoscope apparatus according to Claim 1, wherein the light amount control means (29) is configured to control to decrease the amount of the light in the red wavelength range.

3. The endoscope apparatus according to Claim 2, wherein the light amount control means (29) further includes a control function to increase the amount of the light in the red wavelength range, and when performing the control to increase the light amount, the signal processing means (5, 5B, 5C) performs signal processing to decrease the luminance level of the red color signal that corresponds to the red wavelength range in picking up an image under the illumination light.

4. The endoscope apparatus according to Claim 1, wherein, when the light amount control means (29) controls the illumination light such that substantially no light in the red wavelength range is emitted and the illumination light includes lights in green and blue wavelength ranges other than the light in the red wavelength range, the signal processing means (5, 5B, 5C) is configured to generate a red color signal that corresponds to the red wavelength range from at least one of green and blue color signals that correspond to the green and blue wavelength ranges, respectively.

5. The endoscope apparatus according to Claim 1, wherein the signal processing means (5, 5B, 5C) includes detecting means (71) configured to detect whether or not a predetermined value or more of pixels of an image signal picked up of the observation target region are blue so as to determine whether or not the photosensitizing substance is administered to the observation target region, and the light amount control means (29) is configured to perform the light amount control according to a detection output of the detecting means (71).

6. The endoscope apparatus according to Claim 1,
wherein the signal processing means (5, 5B, 5C) includes detecting means (71) configured to detect when the photosensitizing substance (17) is sprayed on the observation target, and
wherein the light amount control means (29) is configured to decrease the amount of light in a red wavelength range in the illumination light according to a detection output of the detecting means (71).

7. The endoscope apparatus according to Claim 1, wherein the signal processing means (5, 5B, 5C), in response to a light amount change of decreasing the amount of the light in the red wavelength range by the light amount control means (29), performs signal processing to maintain a white balance state that is substantially the same as before the light amount change.

8. The endoscope apparatus according to Claim 3, wherein the signal processing means (5, 5B, 5C), in response to a light amount change of increasing the amount of the light in the red wavelength range by the light amount control means (29), performs signal processing to maintain a white balance state that is substantially the same as before the light amount change.

9. The endoscope apparatus according to Claim 1, wherein the illumination means (4) emits in a time divisional manner illumination lights in a plurality of wavelength ranges different to one another covering a visible range.

10. The endoscope apparatus according to Claim 1, wherein the illumination means (4) emits white light covering a visible range.

11. An endoscope apparatus according to one of claims 4 to 10:
wherein the light amount control means is configured to control the amount of light in the red wavelength range by at least decreasing the amount of light in a red wavelength range in the illumination light; and
wherein the signal processing means is configured to perform the signal processing to change the luminance level of the red color signal by increasing the luminance level of the red color signal.

12. The endoscope apparatus according to Claim 11 in combination with one of claims 5 to 6, wherein the light amount control means (29) is configured to allow the decrease of the amount of light in the red wavelength in response to the detecting means (71) to be cancelled, and to allow manual increase of the amount of light in the red wavelength region.

13. The endoscope apparatus according to Claim 11 in combination with claim 8, wherein the illumination means (4) includes a rotary filter (25, 53, 62a-c) that emits the illumination lights in the plurality of wavelength ranges in a time divisional manner.

14. The endoscope apparatus according to any preceding claim, wherein the photosensitizing substance (17) is methylene blue.

## Patentansprüche

1. Endoskopische Vorrichtung (1B) mit
einem Endoskop (3B), das einen Einführbereich (8) enthält, der in einen lebenden Körper eingeführt werden kann; und
einem Beleuchtungsmittel (4), das so ausgebildet ist, dass es Beleuchtungslicht zu der Seite eines Beobachtungsobjektbereichs in dem lebenden Körper aussendet;
**dadurch gekennzeichnet, dass** sie ferner umfasst:
ein Lichtmengen-Steuerungsmittel (29), das so ausgebildet ist, dass es eine Lichtmenge im Bereich einer roten Wellenlänge in dem Beleuchtungslicht steuert, welche die Erzeugung von reaktiven Sauerstoffspezies durch eine fotosensibilisierende Substanz (17) bewirkt; und
ein Signalverarbeitungsmittel (5, 5B, 5C), welches so ausgebildet ist, dass es in Reaktion auf die Steuerung der Lichtmenge im Bereich der roten Wellenlänge eine Signalverarbeitung durchführt, um den Luminanzgrad eines roten Farbsignals, welches einem Bereich der roten Wellenlänge entspricht, beim Aufnehmen eines Bildes unter dem Beleuchtungslicht zu ändern.

2. Endoskopische Vorrichtung nach Anspruch 1, wobei das Lichtmengen-Steuerungsmittel (29) so ausgebildet ist, dass es eine Verringerung der Lichtmenge im Bereich der roten Wellenlänge steuert.

3. Endoskopische Vorrichtung nach Anspruch 2, wobei das Lichtmengen-Steuerungsmittel (29) ferner eine Steuerungsfunktion zum Steigern der Lichtmenge im Bereich der roten Wellenlänge enthält und, wenn die Steuerung zum Steigern der Lichtmenge ausgeführt wird, das Signalverarbeitungsmittel (5, 5B, 5C) eine Signalverarbeitung durchführt, um den Luminanzgrad des roten Farbsignals, das dem Bereich der roten Wellenlänge entspricht, beim Aufnehmen eines Bildes unter dem Beleuchtungslicht zu verringern.

4. Endoskopische Vorrichtung nach Anspruch 1, wobei, wenn das Lichtmengen-Steuerungsmittel (29) das Beleuchtungslicht so steuert, dass im Wesentlichen kein Licht im Bereich der roten Wellenlänge ausgesendet wird und das Beleuchtungslicht Licht in den Bereichen der grünen und blauen Wellenlängen mit Ausnahme von Licht im Bereich der roten Wellenlänge enthält, das Signalverarbeitungsmittel (5, 5B, 5C) so ausgebildet ist, dass es aus grünen und/oder blauen Farbsignalen, die jeweils den Bereichen der grünen und blauen Wellenlänge entsprechen, ein rotes Farbsignal erzeugt, das dem Bereich der roten Wellenlänge entspricht.

5. Endoskopische Vorrichtung nach Anspruch 1, wobei das Signalverarbeitungsmittel (5, 5B, 5C) ein Erkennungsmittel (71) enthält, welches so ausgebildet ist, dass es erkennt, ob eine vorgegebene oder größere Menge von Pixeln eines Bildsignals, das aus dem Beobachtungsobjektbereich aufgenommen wurde, blau ist oder nicht, um so zu bestimmen, ob die fotosensibilisierende Substanz an den Beobachtungsobjektbereich ausgegeben wird oder nicht, und das Lichtmengen-Steuerungsmittel (29) so ausgebildet ist, dass es die Lichtmengensteuerung gemäß eines Erkennungsergebnisses des Erkennungsmittels (71) steuert.

6. Endoskopische Vorrichtung nach Anspruch 1,
wobei das Signalverarbeitungsmittel (5, 5B, 5C) ein Erkennungsmittel (71) enthält, welches so ausgebildet ist, dass es erkennt, wenn die fotosensibilisierende Substanz (17) auf das Beobachtungsobjekt gesprüht wird, und
wobei das Lichtmengen-Steuerungsmittel (29) so ausgebildet ist, dass es die Lichtmenge im Bereich der roten Wellenlänge im Beleuchtungslicht gemäß eines Erkennungsergebnisses des Erkennungsmittels (71) verringert.

7. Endoskopische Vorrichtung nach Anspruch 1, wobei das Signalverarbeitungsmittel (5, 5B, 5C) in Reaktion auf eine Lichtmengenänderung des Verringerns der Lichtmenge im Bereich der roten Wellenlänge durch das Lichtmengen-Steuerungsmittel (29) eine Signalverarbeitung durchführt, um einen Zustand des Weißabgleichs aufrechtzuerhalten, der im Wesentlichen derselbe wie vor der Lichtmengenänderung ist.

8. Endoskopische Vorrichtung nach Anspruch 3, wobei das Signalverarbeitungsmittel (5, 5B, 5C) in Reaktion auf eine Lichtmengenänderung des Steigerns der Lichtmenge im Bereich der roten Wellenlänge durch das Lichtmengen-Steuerungsmittel (29) eine Signalverarbeitung durchführt, um einen Zustand des Weißabgleichs aufrechtzuerhalten, der im Wesentlichen derselbe wie vor der Lichtmengenänderung ist.

9. Endoskopische Vorrichtung nach Anspruch 1, wobei das Beleuchtungsmittel (4) zeitbereichsweise Beleuchtungslicht in mehreren Bereichen von Wellenlängen aussendet, die sich voneinander unterscheiden und einen sichtbaren Bereich abdecken.

10. Endoskopische Vorrichtung nach Anspruch 1, wobei das Beleuchtungsmittel (4) weißes Licht aussendet, das einen sichtbaren Bereich abdeckt.

11. Endoskopische Vorrichtung nach einem der Ansprüche 4 bis 10,
wobei das Lichtmengen-Steuerungsmittel so ausgebildet ist, dass es die Lichtmenge im Bereich der roten Wellenlänge zumindest durch Verringern der Lichtmenge in einem Bereich der roten Wellenlänge im Beleuchtungslicht steuert; und
wobei das Signalverarbeitungsmittel so ausgebildet ist, dass es die Signalverarbeitung ausführt, um den Luminanzgrad des roten Farbsignals durch Steigern des Luminanzgrads des roten Farbsignals zu verändern.

12. Endoskopische Vorrichtung nach Anspruch 11 in Verbindung mit einem der Ansprüche 5 bis 6, wobei das Lichtmengen-Steuerungsmittel (29) so ausgebildet ist, dass es gestattet, dass die Verringerung der Lichtmenge in der roten Wellenlänge in Reaktion auf das Erkennungsmittel (71) aufgehoben wird, und dass es eine manuelle Steigerung der Lichtmenge im Bereich der roten Wellenlänge zulässt.

13. Endoskopische Vorrichtung nach Anspruch 11 in Verbindung mit Anspruch 8, wobei das Beleuchtungsmittel (4) einen Drehfilter (25, 53, 62a-c) enthält, der das Beleuchtungslicht zeitbereichsweise in den mehreren Bereichen von Wellenlängen aussendet.

14. Endoskopische Vorrichtung nach einem der vorhergehenden Ansprüche, wobei die fotosensibilisierende Substanz (17) Methylenblau ist.

## Revendications

1. Appareil d'endoscope (1B) comprenant :
un endoscope (3B) comprenant une partie d'insertion (8) qui peut être insérée dans un corps vivant ; et
un moyen d'éclairage (4) qui est configuré pour émettre une lumière d'éclairage vers un côté de région cible d'observation dans le corps vivant ;
**caractérisé en ce qu'**il comprend en outre :
un moyen de commande de quantité de lumière (29) configuré pour commander une quantité de lumière dans une plage de longueurs d'onde du rouge dans la lumière d'éclairage qui provoque la production d'espèces d'oxygène réactif par un photosensibilisant (17) ; et
un moyen de traitement de signal (5, 5B, 5C) qui, en réponse à la commande de quantité de lumière de la plage de longueurs d'onde du rouge, est configuré pour réaliser un traitement de signal pour changer un niveau de luminance d'un signal de couleur rouge qui correspond à une plage de longueurs d'onde du rouge en capturant une image sous la lumière d'éclairage.

2. Appareil d'endoscope selon la revendication 1, dans lequel le moyen de commande de quantité de lumière (29) est configuré pour commander la diminution de la quantité de lumière dans la plage de longueurs d'onde du rouge.

3. Appareil d'endoscope selon la revendication 2, dans lequel le moyen de commande de quantité de lumière (29) possède en outre une fonction de commande pour augmenter la quantité de lumière dans la plage de longueurs d'onde du rouge, et lors de l'exécution de la commande d'augmentation de la quantité de lumière, le moyen de traitement de signal (5, 5B, 5C) réalise un traitement de signal pour diminuer le niveau de luminance du signal de couleur rouge qui correspond à la plage de longueurs d'onde du rouge en capturant une image sous la lumière d'éclairage.

4. Appareil d'endoscope selon la revendication 1, dans lequel, lorsque le moyen de commande de quantité de lumière (29) commande la lumière d'éclairage d'une manière telle que pratiquement aucune lumière dans la plage de longueurs d'onde du rouge n'est émise et que la lumière d'éclairage comprend des lumières dans les plages des longueurs d'onde du vert et du bleu autres que la lumière dans la plage de longueurs d'onde du rouge, le moyen de traitement de signal (5, 5B, 5C) est configuré pour générer un signal de couleur rouge qui correspond à la plage de longueurs d'onde du rouge à partir d'au moins l'un parmi les signaux de couleur du vert et du bleu qui correspondent aux plages des longueurs d'onde du vert et du bleu, respectivement.

5. Appareil d'endoscope selon la revendication 1, dans lequel le moyen de traitement de signal (5, 5B, 5C) comprend un moyen de détection (71) configuré pour détecter si oui ou non une valeur prédéterminée ou plus de pixels d'un signal d'image capturée de la région cible d'observation sont bleus de façon à déterminer si oui ou non le photosensibilisant est administré sur la région cible d'observation, et le moyen de commande de quantité de lumière (29) est configuré pour réaliser la commande de quantité de lumière conformément à une sortie de détection du moyen de détection (71).

6. Appareil d'endoscope selon la revendication 1,
dans lequel le moyen de traitement de signal (5, 5B, 5C) comprend un moyen de détection (71) configuré pour détecter lorsque le photosensibilisant (17) est pulvérisé sur la cible d'observation, et
dans lequel le moyen de commande de quantité de lumière (29) est configuré pour diminuer la quantité de lumière dans une plage de longueurs d'onde du rouge dans la lumière d'éclairage conformément à une sortie de détection du moyen de détection (71).

7. Appareil d'endoscope selon la revendication 1, dans lequel le moyen de traitement de signal (5, 5B, 5C), en réponse à un changement de quantité de lumière consistant à diminuer la quantité de la lumière dans la plage de longueurs d'onde du rouge par le moyen de commande de quantité de lumière (29), réalise un traitement de signal pour maintenir un état d'équilibre des blancs qui est sensiblement le même qu'avant le changement de quantité de lumière.

8. Appareil d'endoscope selon la revendication 3, dans lequel le moyen de traitement de signal (5, 5B, 5C), en réponse à un changement de quantité de lumière consistant à augmenter la quantité de la lumière dans la plage de longueurs d'onde du rouge par le moyen de commande de quantité de lumière (29), réalise un traitement de signal pour maintenir un état d'équilibre des blancs qui est sensiblement le même qu'avant le changement de quantité de lumière.

9. Appareil d'endoscope selon la revendication 1, dans lequel le moyen d'éclairage (4) émet des lumières d'éclairage de manière divisée dans le temps dans une pluralité de plages de longueurs d'onde différentes les unes des autres couvrant une plage visible.

10. Appareil d'endoscope selon la revendication 1, dans lequel le moyen d'éclairage (4) émet une lumière blanche couvrant une plage visible.

11. Appareil d'endoscope selon l'une quelconque des revendications 4 à 10 :
dans lequel le moyen de commande de quantité de lumière est configuré pour commander la quantité de lumière dans la plage de longueurs d'onde du rouge au moins en diminuant la quantité de lumière dans la plage de longueurs d'onde du rouge dans la lumière d'éclairage ; et
dans lequel le moyen de traitement de signal est configuré pour réaliser le traitement de signal pour changer le niveau de luminance du signal de couleur rouge en augmentant le niveau de luminance du signal de couleur rouge.

12. Appareil d'endoscope selon la revendication 11 en combinaison avec l'une quelconque parmi les revendications 5 à 6, dans lequel le moyen de commande de quantité de lumière (29) est configuré pour permettre la diminution de la quantité de lumière dans la longueur d'onde du rouge en réponse au moyen de détection (71) à annuler, et pour permettre l'augmentation manuelle de la quantité de lumière dans la région de longueurs d'onde du rouge.

13. Appareil d'endoscope selon la revendication 11 en combinaison avec la revendication 8, dans lequel le moyen d'éclairage (4) comprend un filtre rotatif (25, 53, 62a à c) qui émet les lumières d'éclairage dans la pluralité de plages de longueurs d'onde de manière divisée dans le temps.

14. Appareil d'endoscope selon l'une quelconque des revendications précédentes, dans lequel le photosensibilisant (17) est du bleu de méthylène.
